# EUROPEAN PATENT SPECIFICATION

(11) **EP 1 104 482 B1**
(45) Date of publication and mention of the grant of the patent: **19.10.2005**
(21) Application number: 99939723.5
(22) Date of filing: 10.08.1999
(51) Int. Cl.: C12N 15/867, C12N 15/62

(54) **CELL TYPE SPECIFIC GENE TRANSFER USING RETROVIRAL VECTORS CONTAINING ANTIBODY-ENVELOPE FUSION PROTEINS AND WILD-TYPE ENVELOPE FUSION PROTEINS**
ZELLTYP SPECIFISCHER GENTRANSFER DER RETROVIRALEN VEKTOREN DIE ANTIKÖRPER-HÜLLEN FUSIONSPROTEINE UND WILDTYP-HÜLLEN FUSIONSPROTEINE ENTHALTEN
TRANSFERT DE GENES A SPECIFICITE DE TYPE CELLULAIRE UTILISANT DES VECTEURS RETROVIRAUX CONTENANT DES PROTEINES DE FUSION ANTICORPS ENVELOPPE ET PROTEINES DE FUSION D'ENVELOPPE DE TYPE SAUVAGE

(30) Priority: 17.08.1998 US 135121
(43) Date of publication of application: 06.06.2001
(73) Proprietor: THOMAS JEFFERSON UNIVERSITY, Philadelphia, PA 19107-6799 (US)
(72) Inventor: DORNBURG, Ralph C., Philadelphia, PA 19128 (US)
(74) Representative: Pilkington, Stephanie Joan
(86) International application number: PCT/US1999/018141
(87) International publication number: WO 2000/009730

(56) References cited:
- WO-A-95/23846
- CHU T -H T ET AL: "RETROVIRAL VECTOR PARTICLES DISPLAYING THE ANTIGEN-BINDING SITE OF AN ANTIBODY ENABLE CELL-TYPE-SPECIFIC GENE TRANSFER" JOURNAL OF VIROLOGY,US,THE AMERICAN SOCIETY FOR MICROBIOLOGY, vol. 69, no. 4, 1 April 1995 (1995-04-01), pages 2659-2663, XP000615436 ISSN: 0022-538X
- CHU T-H T & DORNBURG R: "Towars highly efficientcell-type-specific gene transfer with retroviral vectors displaying single-chain antibodies" JOURNAL OF VIROLOGY,US,THE AMERICAN SOCIETY FOR MICROBIOLOGY, vol. 71, no. 1, January 1997 (1997-01), pages 720-725-725, XP002104908 ISSN: 0022-538X

## Description

### Field of the Invention

This invention relates to use of retroviral vector particles having target cell specificity in the manufacture of a medicament. The retroviral vector particles comprise a retroviral vector having a chimeric envelope protein consisting of an antigen binding site of an antibody or-another peptide fused to the envelope protein of the retroviral vector. The antigen binding site or the other peptide replaces or disrupts the natural viral receptor binding site. The resulting chimeric envelope is referred to as the "targeting envelope". This invention relates to the use of retroviral vectors that contain not only the targeting envelope but also wild-type envelope protein. The presence of wild-type envelope in addition to the targeting envelope acts as a helper molecule by supplying a fully functional membrane fusion domain, which may be impaired in targeting envelopes. This helper function enables and/or enhances infection of cells that do not contain a receptor for the wild-type envelope but do contain a receptor for the binding for the targeting molecule. We also provide a method for preparing the retroviral particles and for using the retroviral vectors to introduce genes into vertebrate cells.

### Description of the Background

The disclosures referred to herein illustrate the background of the invention and provide additional detail with respect to its practice. For convenience, the disclosures are referenced in the following text and respectively grouped in the appended bibliography.

Retroviral vectors are the most efficient tools to introduce genes into vertebrate cells. Clinical experiments have been conducted to use retrovirus vectors to cure a genetic disease in humans (adenosine deaminase (ADA) deficiency). Besides correcting inborn errors of metabolism, gene therapy is also being tested in clinical trials to cure cancer and various other diseases (Science 1992, Vol. 258, pp. 744-746).

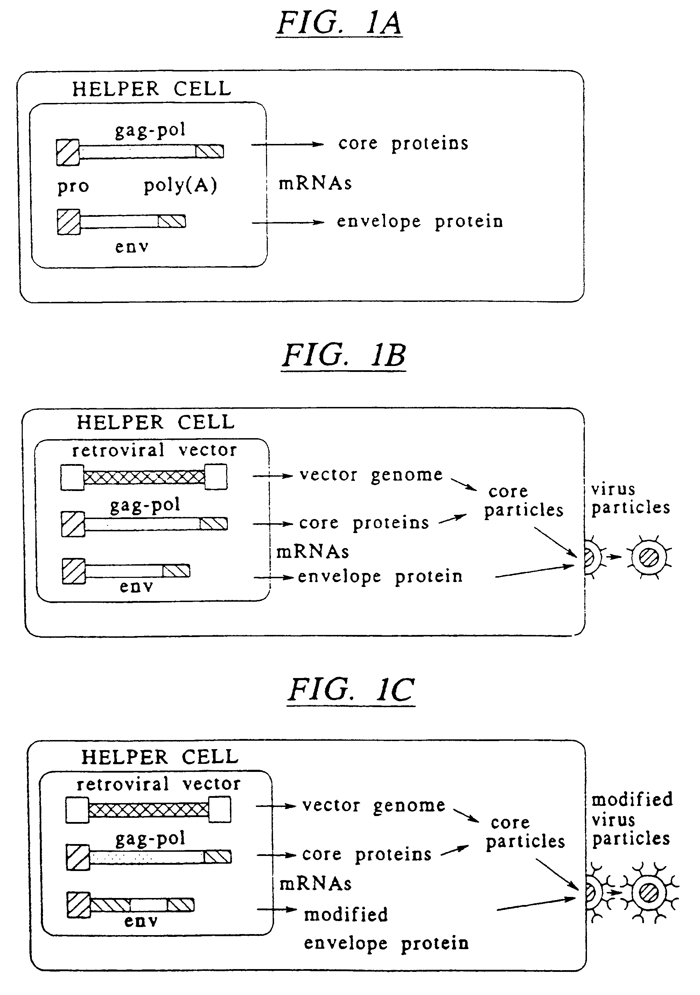

Retroviral vectors are basically retroviral particles that contain a genome in which all viral protein coding sequences have been replaced with the gene(s) of interest. As a result, such viruses cannot further replicate after one round of infection. Retroviral vector particles are produced by helper cells (Figure 1). Such helper cells are cell lines that contain plasmid constructs, which express all retroviral proteins necessary for replication. After transfection of the vector genome into such helper cells, the vector genome is encapsidated into virus particles (due the presence of specific encapsidation sequences). Virus particles are released from the helper cell carrying a genome containing only the gene(s) of interest (Figure 1). In the last decade, several retroviral vector systems derived from chicken or murine retroviruses, have been developed for the expression of various genes (for reviews see Temin. 1987; Gilboa, 1990).

Retroviral vectors have several limitations. Besides the limited genome size that can be encapsidated into viral particles, the most limiting factor for the application of retroviral vectors is the restricted host range of the vector particle. Some retroviruses can only infect cells of one species (ecotropic retroviruses) or even only one cell-type of one species (*e.g*.. HIV). Other retroviruses have a very broad host range and can infect many different types of tissues of many different species (amphotropic retroviruses).

The initial step of retroviral infection is the binding of the viral envelope (*env*) glycoprotein to specific cell membrane receptors, the nature of which is unknown for most retroviruses. However, the interaction of the viral *env* protein with the cell surface receptor is very specific and determines cell-type specificity of a particular virus (Weiss et al.. 1985). The envelope protein of all known retroviruses is made up of two associated peptides, (*e.g.*, gp70 and p20(E) in SNV). These peptides are derived by proteolytic cleavage from the same precursor (gPR90env) encoded by the retroviral *env* gene. One peptide p20(E), also termed TM, anchors the protein in the membrane of the virus and, as shown with HIV, mediates the fusion of the virus and cell membranes. The second peptide gp70, also termed SU, mediates the binding of the virus to its receptor and, therefore, determines the host range (Weiss et al., 1985; Varmus and Brown, 1989).

Data obtained with several retroviruses indicate that the retroviral envelope protein forms trimers or tetramers. The formation of trimers appears to be mediated by the TM peptide (reviewed in Hunter, E. et al., 1990). Targeting envelopes retain TM in order to (i.) maintain a membrane fusion function and (ii) maintain oligomerization, However, since X-ray pictures are not available, it is unclear whether or to what degree the construction of targeting-molecules impaired the structure of the membrane fusion domain.

### Brief Description of the Figures

Figure 1 is a diagram illustrating helper cells expressing retroviral proteins: (A) Helper cells are made by the transfection of plasmids expressing all retroviral proteins necessary to form infectious virus particles; (B) After transfection of the retroviral vector, the vector RNA genome is encapsidated into core structures; (C) Helper cells that contain a plasmid express a modified envelope gene.
Figure 2 is a diagram illustrating plasmids expressing mutant envelope genes of spleen necrosis virus (SNV).
Figure 3 shows the sequence of the single chain antibody gene (scFv) against the hapten DNP.

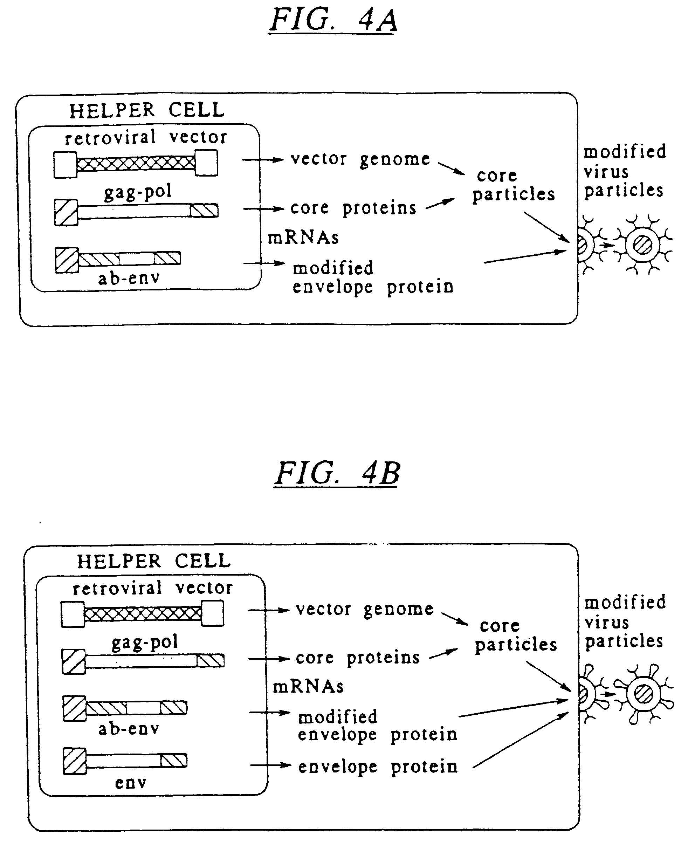

Figure 4 is a diagram illustrating helper cells expressing targeting envelopes plus wild-type envelopes. Such helper cells are made by the transfection of plasmids expressing the corresponding proteins: (A) A helper cell expressing all retroviral proteins necessary to form (1) retroviral core proteins and (2) targeting envelope; (B) Helper cells that contain targeting plus wild-type envelope are made by transfecting plasmids expressing genes encoding such proteins. After transfection of the retroviral vector that has the gene of interest, the retroviral vector RNA genome is encapsidated into retroviral vector particles displaying the envelope.
Figure 5 is a diagram of a eucaryotic gene expression vector construct. The gene expression vector was derived from a similar vector described by Sheay, W. et al., 1993.
Figure 6 is a diagram illustrating plasmids expressing spleen necrosis virus. SNV, core structure proteins, wild-type envelope proteins, and various targeting envelope proteins.
Figure 7 is a restriction map of pRSV-scFvN29-gamma expressing the anti-Her2neu single chain antibody gene including the authentic hydrophobic leader sequence.
Figure 8 shows the sequence of the anti-Her2neu single chain antibody.
Figure 9 is a restriction map of pTC53. PCR products of single chain antibody genes can be cloned into the Nael site and the resulting construct expresses a chimeric envelope, which is transported through the ER and displayed on the surface of SNV derived retroviral vector particles.

### Summary of the Invention

The present invention pertains to the use in preparing a medicament of a retroviral vector particle having defined target cell specificity mediated by the nature of the targeting envelope which can be a chimeric protein consisting of an antigen binding site of an antibody or another peptide that binds to a specific cell surface structure (*e.g*., the receptor binding domain of another virus) fused to carboxy terminal parts of the retroviral envelope protein. The targeting envelope mediates the first step of retroviral infection, which is the binding of the virus to a specific cell-surface receptor. The present invention also pertains to the use in preparing a medicament of retroviral particles that contain a wild-type envelope in addition to the targeting envelope. The presence of the wild-type envelope serves to act as a helper molecule to improve or supplement a functional membrane fusion domain. Using target cells that do not contain a receptor for the wild-type envelope (*e.g*., SNV is not infectious for human cells), the wild-type envelope is only involved in the second step of retroviral infection, which is the efficient fusion of the viral and the cellular membranes. We also discuss the construction of retroviral vectors particles containing a wild-type envelope in addition to a targeting envelope which can compensate for the loss of infectivity observed with retroviral particles that contain targeting envelopes alone.

In one embodiment, the present invention pertains to use of a retroviral vector having an envelope protein wherein a viral receptor portion is at least partially defined by a single chain antibody and said viral receptor portion binds to a selected antigen in the manufacture of a medicament for use in infecting in vivo a cell having the said selected antigen wherein the said medicament is for contacting said retroviral vector in vivo with the cell such that said viral particle or a portion thereof is internalized into the cell, and the cell having the selected antigen is infected by said retroviral vector.

In another embodiment, the present invention pertains to use of a retroviral vector having a targeting peptide fused to the envelope protein of said retroviral vector to form a targeting envelope in the manufacture of a medicament for infecting in vivo a cell having a selected antigen wherein the said medicament is for contacting said retroviral vector in vivo with the cell such that said viral particle or a portion thereof is internalized into the cell, and the cell having the selected antigen is infected by said retroviral vector.

In a further embodiment, the present invention pertains to use of a retroviral vector particle having target cell specificity which comprises a retroviral vector having an antigen binding site of an antibody fused to the envelope protein of the retroviral vector, wherein the antigen binding side of the antibody replaces the natural viral receptor binding site, in the manufacture of a medicament for a cell type specific method for introducing in vivo genes into vertebrate cells using retroviral vectors.

### Detailed Description of the Invention

### Targeting Envelope

This invention relates to the use in the manufacture of a medicament of retroviral vector particles having target cell specificity, as set out in the claims. The retroviral vector particles comprise a retroviral vector having a chimeric envelope protein consisting of an antigen binding site of an antibody or another peptide fused to the envelope protein of the retroviral vector. The antigen binding site or the other peptide replaces or disrupts the natural viral receptor binding site. The resulting chimeric envelope is referred to as the "targeting envelope". This invention relates to the use of retroviral vectors that contain not only the targeting envelope but also wild-type envelope protein. The presence of wild-type envelope in addition to the targeting envelope acts as a helper molecule by supplying a fully functional membrane fusion domain which may be impaired in targeting envelopes. This helper function enables and/or enhances infection of cells that do not contain a receptor for the wild-type envelope but do contain a receptor for the binding of the targeting molecule. We also provide a method for preparing the retroviral particles and for using the retroviral vectors to introduce genes into vertebrate cells.

To alter the host range of a vector particle, retroviral vector particles may be constructed that contain modified envelope proteins that recognize only a cell surface structure (receptor) specific for the target cell of interest. Proteins known to recognize specific structures of proteins are antibody molecules. Hence, to make a retroviral vector particle specific for a cell-type of interest, the viral receptor binding peptide may be replaced with an antigen binding site of an antibody molecule. To test whether vector particles containing such antigen binding sites are competent for infection, model systems were developed using an antigen binding peptide of an antibody against the hapten dinitrophenol (DNP) fused to envelope gene of spleen necrosis virus (SNV).

The use of the anti-hapten (anti-DNP) antibody has many advantages. (1) The interaction of this antigen with the antibody is well characterized (Davies and Metzger, 1983). (2) The hapten is easily available. (3) A large variety of cells (which cannot be infected with wild-type vector particles) can be conjugated with this hapten. DNP conjugated cells bind antibodies directed against this hapten. Thus, the hapten may mimic the (abundant) presence of a receptor for the chimeric vector particle. (4) Anti-hapten antibodies are frequently internalized by the cell. Thus, the construction of chimeric envelope proteins will destroy the membrane fusion domain of TM. This property may compensate for this loss of function. (5) An *in vitro* binding assay can be easily established to test for virus particle formation and binding of such viruses to DNP. (6)

### Wild-Type Envelope

This invention relates to the use of retroviral particles having a target cell specificity. The retroviral vector particles comprise a retroviral vector having a targeting envelope which mediates the binding of the retroviral vector particle to a cell surface receptor of the target cell. This binding is very specific and determines the host range and cell-type specificity. The particles also have a wild type envelope. Using target cells that do not contain a viable receptor for the wild type envelope, the function of the wild-type envelope is only to supply a fully functional membrane fusion domain. We also provide the method for preparing the retroviral vector particles and a method for using the retroviral vectors to introduce genes into vertebrate cells.

Retroviral vectors derived from spleen necrosis virus containing wild-type envelope alone cannot infect human or hamster cells. In these infectivity studies, retroviral particles harvested from DSN cells were used (Dougherty, J.P. and Temin, H.M. 1989) to infect human HeLa and Col-1, as well as hamster CHTG (ret. 1) cells (Tables 1 and 2). DSN cells are standard retroviral packaging cells containing a plasmid expressing the retroviral core proteins and another plasmid expressing wild-type envelope (Dougherty, J.P. and Ternin, H.M. 1989).

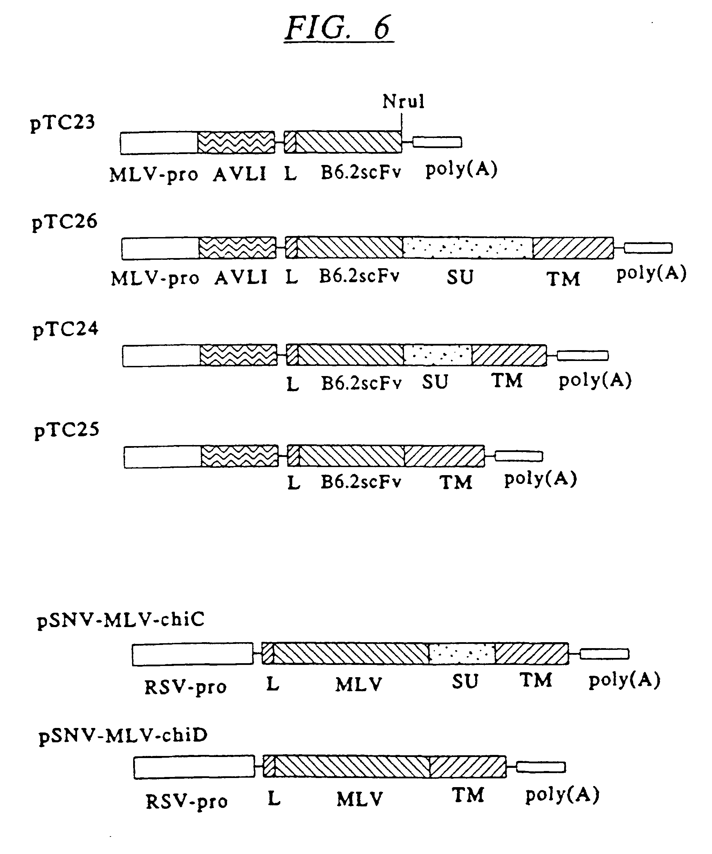

To introduce genes into such cells using SNV retroviral vector particles, two different approaches were made using different targeting envelopes in combination with and without additional wild-type envelope.
1. Targeting of human cancer cells (HeLa and Col-1) with SNV retroviral vectors. The antigen binding site of an antibody directed against the hapten DNP was used. In the experiments described below, the antigen binding site used in the targeting envelope was derived from an antibody (termed B62, Bird, R.E. et al., 1988 and Colcher, D. et al., 1990) directed against a cell-surface protein expressed on various human cancers (e.g.. HeLa and Col-1 cells, Bird, R.E. et al., 1988 and Colcher, D. et al., 1990). The gene constructs (Figure 6) for the expression of the targeting envelope are similar to that described above. In particular, in two constructs (Figure 6, pTC24 and pTC25), the antibody moiety was fused to the same position of the SNV envelope gene as the anti-DNP antibody described below (for more details, see below: Material and Methods). To test whether the addition of a fully functional membrane fusion domain (provided by wild-type envelope) would increase the efficiency of infection, helper cells expressing retroviral core proteins, wild-type envelope, and the targeting envelope were developed (Figure 4). Virus was harvested from such helper cells and subjected to infectivity studies.
2. Targeting CHTG cells that express a receptor for ecotropic murine leukemia virus. To test whether retroviral particles derived from SNV displaying targeting molecules other than antigen binding sites of an antibody are infectious, targeting envelopes were constructed that contained the receptor binding peptide of another virus (murine leukemia virus) fused to the envelope of SNV. Infectivity of virus particles displaying such targeting envelopes with and without wild-type envelope was tested.

### Examples

### Targeting Envelope

### Materials and Methods

### Construction of Antibody-Envelope Fusion Genes

The gene coding for the envelope protein of spleen necrosis virus (SNV) does not contain suitable restriction enzyme sites to enable the construction of antibody-envelope fusion genes. Thus, point mutations were introduced (by site directed mutagenesis) in the SNV *env* gene at different locations to create restriction enzyme recognition sites. For this purpose, the SNV *env* gene (HindIII-Sacl fragment) was subcloned into pSelect (a vector specifically designed for site directed mutagenesis). Restriction sites for enzymes that create blunt ends were introduced in such a way that the restriction enzymes cut between two codons. Following consistently this strategy, all mutants can be used to create deletions, insertions, and fusions in any combination without altering the reading frame. Further, restriction enzyme sites were nested between regions coding for hydrophobic and hydrophilic domains. It was hypothesized that the deletion of a certain domain(s) would not interfere with the proper folding of the following domain. This hypothesis is based on the finding that many proteins in evolution arose by exon shuffling of functional domains.

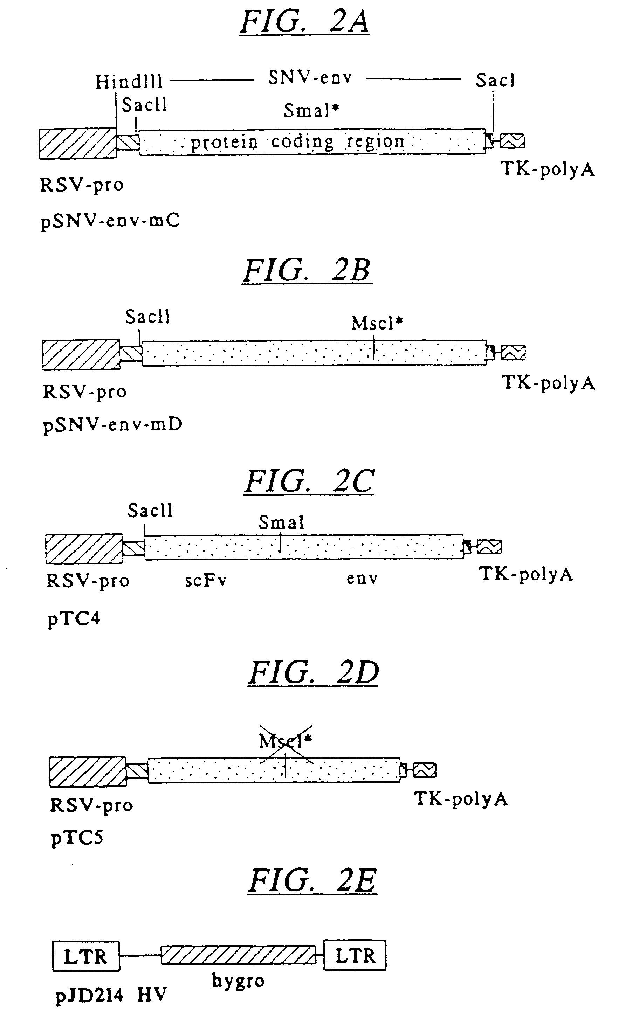

Some mutant envelopes that have been made are shown in Figure 2. pSNV-env-mC (Figure 2a) contains a new restriction enzyme site located between a hydrophobic and a hydrophilic peptide domain. In this mutant, the change in the nucleotide sequence does not alter the amino acid sequence. Thus, pSNV-env-mC can be considered as a positive control. pSNV-env-mD contains a new restriction enzyme site within the cleavage site of the envelope precursor. The introduction of the mutation also altered the amino acid sequence destroying the common motive found in all cleavage sites of all retroviruses investigated. Thus, it was expected that the resulting envelope precursor would not be cleaved, and, therefore, would not to give rise to infectious virus particles. Mutated *env* genes were inserted into pHB3, a eucaryotic gene expression vector (Figure 2).

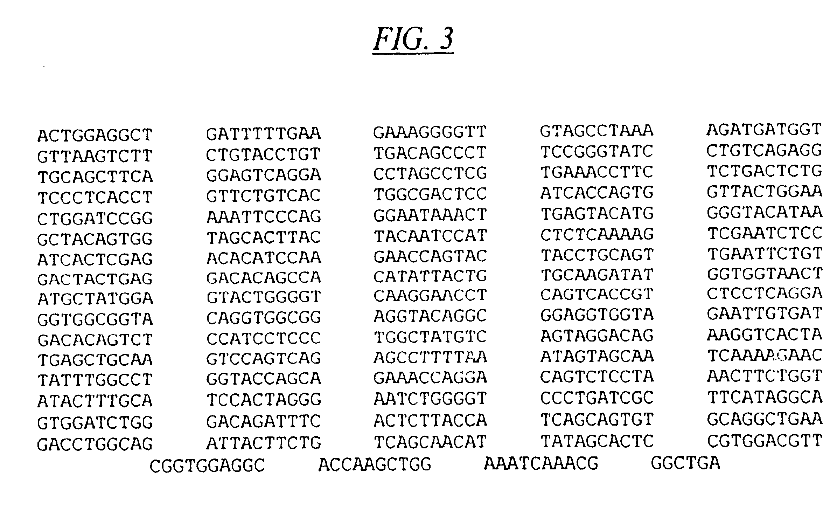

The genes coding for the heavy and the light chain of an antibody against DNP have been kindly provided by Dr. Ogawa (Scripps Clinic, La Jolla, CA). The genes were sequenced and published (Riley et al., 1986). Using PCR technology as described (Whitlow and Filpula, 1990), a single chain antibody gene was constructed including the signal peptide against DNP. The PCR product was cloned into the Smal site of pBluescript. DNA sequencing confirmed the successful combination of the two gene segments coding for the variable regions of the antigen binding peptide. The complete sequence of the anti-DNP scFv gene is given in Figure 3. A SacII (located in the polylinker of pBluescript) to SmaI (located in the 3' PCR primer) fragment was inserted into eucaryotic expression vectors replacing amino terminal parts of the envelope gene as follows: in PTC4, the SacII (located upstream of the ATG codon of the *env* gene) to SmaI fragment of *env* was replaced with the scFv gene' in pTC5 the SacII to the MscI fragment of *env* was replaced with the scFv gene (Figure 2C and 2D, respectively). After cloning, the antibody-envelope junctions were sequenced to verify the maintenance of the correct reading frame of the chimeric gene.

### Binding Assays

The *in vitro* binding assays were performed in the following manner. DNP was conjugated to BSA (DNP-BSA was used to raise the initial antibodies from which the scFv genes have been derived). DNP-BSA was coupled to activated Sepharose following the protocol recommended by the supplier (Sigma). An Elisa assay with an anti-DNP antibody (kindly provided by Dr. S. Pestka) confirmed the successful coupling reaction. 100 ml of tissue culture supernatant medium was incubated with 50 ml of DNP-BSA-Sepharose for 30 minutes at 7°C. After incubation, the sepharose particles were pelleted by centrifugation in a Qualitron minicentrifuge for 30 seconds. The pellets were rinsed once with PBS. The PBS was removed and reverse transcription assays were performed by adding the reaction to the sepharose pellet. The reverse transcription assay was done using standard procedures; incorporation of 32PdTTP into cDNA was determined by TCA precipitation as described (Schleif and Wensink, 1981)

### Test for Infectivity Of Particles Containing Antibody-Envelope Fusion Proteins

The envelope expression plasmids shown in Figure 2 were transfected into D17 cells (a dog osteosarcoma cell-line) in contransfection with pBR1 and pJD214HY (Figure 2), plasmids expressing the retroviral core proteins, and containing a retroviral vector for the expression of the hygromycin phosphotransferase gene, respectively (see also Figure 1). Cells were selected for hygromycin resistance. After selection for hygromycin resistance, virus was harvested from confluent cell cultures and infectivity assays were performed (see below). Infected target cells were selected for hygromycin resistance (D17 cells were incubated with medium containing 60mg/ml hygromycin, CHO cells with medium containing 250 mg/ml hygromycin). Hygromycin resistant cell colonies indicate infectious virus particles.

Infectivity assays were performed on D17 and CHO cells with and without conjugated DNP. DNP was conjugated to cells as follows: Cells were incubated with 500 ml of a solution containing 1.4 mg/ml DNBS (2, 4,-Dinitrobenzene-sulfonic acid, 2-hydrate, purchased from Kodak) in sodium cocodylate buffer (0.25M) for 3 to 5 minutes at room temperature. The conjugation reaction was stopped by adding 5 ml of medium to the cells.

Infections of non-conjugated cells were performed in the presence of 50 mM polybrene using standard protocols. In the case of DNP conjugated cells, infection was performed without polybrene.

### Wild-Type Envelope

### Material and Methods

### ScA targeting vectors

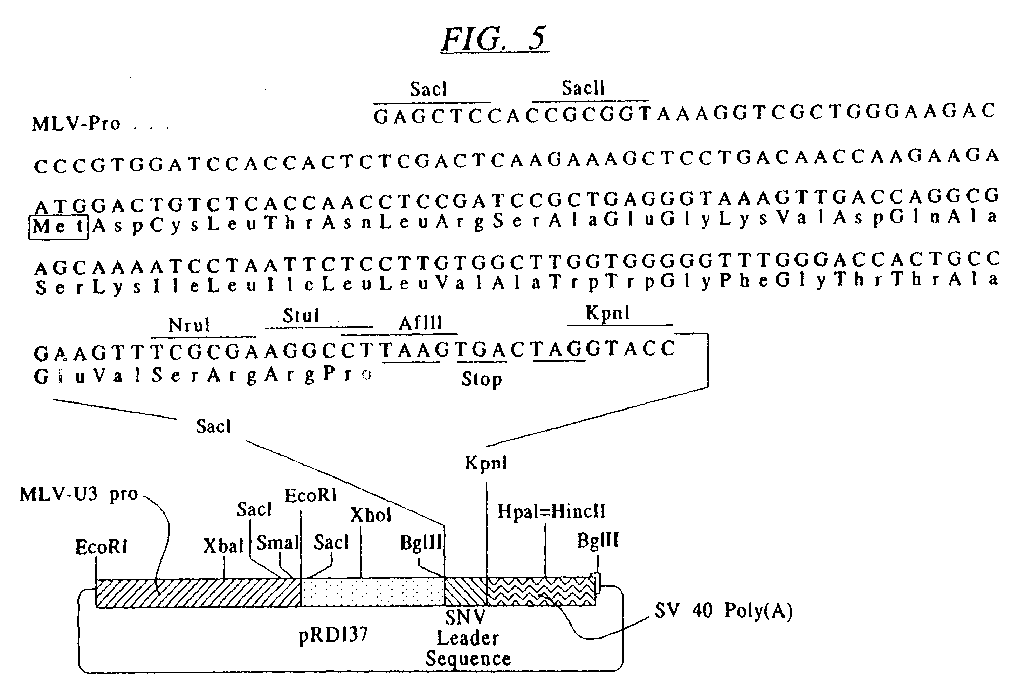

To construct a targeting envelope containing the antigen binding site of an antibody directed against a cell-surface protein expressed on several human tumor cells, the corresponding single chain antibody gene (termed B6.2, Bird, R.E. et al., 1988 and Colcher, D. et al., 1990) made for expression in E.coli was modified in the following way: PCR technology was used to amplify the B6.2 scA gene using the original E.coli expression plasmid as template (Bird, R.E. et al., 1988 and Colcher. D. et al., 1990). The primers used had the following sequence: Primcr A: 5' GGAGCGCTGACGTCGTGATGACCCAGTC 3' Primer B: 5' CCTCGCGATCCACCGCCGGAGACTGTGAGAGTGGTGC 3' The PCR amplification results in a fragment that does not contain the bacterial ompA signal sequence and the stop codons present in the original B6.2 gene (Bird, R.E. et al., 1988 and Colcher, D. et al., 1990). The PCR products were cloned into the Smal site of the pBluescript vector (Strata gene) and sequenced to verify a correct reading frame. The plasmid was termed pTC9. The B6.2 gene was isolated by digesting the pTC9 plasmid with Eco47III plus NruI. The corresponding restriction enzyme recognition sites have been introduced with the primers used for PCR amplification. The B6.2 gene (the Eco47III to Nrul fragment was cloned into pTC13, a gene expression vector (Figure 5). The corresponding vector (termed pTC23) contains the ER transport signal sequence of the SNV envelope protein fused to the B6.2 gene to enable transport through the endoplasmatic reticulum. The cloning reconstituted the NruI site at the 3' end of the B6.2 gene. Carboxy terminal parts of the SNV envelope gene were isolated and fused to the B.2 gene (Nrul site) to give plasmids pTC24, pTC25, and pTG26 (Figure 6). These constructs are very similar to plasmids pTC4 and pTC5 which contain the anti-DNP antibody. In plasmid pTC26, the antibody is fused to codon 168 of the SNV envelope.

### Chimeric SNV-MLV targeting envelope

Targeting envelopes containing the receptor binding peptide of another virus were made as follows: the gene segment of ecotropic murine leukemia virus (a HindIII-BaII fragment comprising almost the complete region coding for the SU peptide, including its ER transport signal sequence, Ott, D., and Rein, A. 1992) was isolated and inserted into the vectors pSNV-env-mC and pSNV-env-mD (pSNV-env-mC and pSNV-env-mD was described in Figure 2) replacing the amino terminal parts of the SNV envelope gene. The resulting constructs are identical to plasmids pTC4 and pTC5, respectively, except that the anti-DNP antibody peptide (anti-DNP scA) is replaced by the receptor binding peptide of ecoMLV (Figure 6, pSNV-MLV-chiC and pSNV-MLV-chi-D, respectively).

### Experimental system

Briefly, helper cells were made as described above by transfecting plasmids expressing retroviral gag-pol proteins, the retroviral targeting envelope, and the wild-type envelope into D17 cells in co-transfection with a selectable marker to obtain helper cell lines containing targeting envelope only or helper cells containing both targeting and wild-type envelope. Infectivity assays were performed on a variety of different cell-lines which included D17 cells, CHTG-cells expressing the ecotropic murine leukemia virus receptor (Albritton, L.M. et al., 1989) and human HeLa and Col-1 cells. Infectivity was determined with a retroviral vector expressing the bacterial beta-galactosidase gene as described (Mikawa, T. et al.).

### Results

### Targeting Envelope

*In vitro* binding assay. The *in vitro* binding assays showed that only cells transfected with pSNV-env-mD produce viral vector particles that contain a chimeric envelope able to bind DNP (see also Table 1).

Infectivity studies. The results of the infectivity experiments are summarized in Table 1. Vector particles containing wild-type envelope (pSNV-env-mC) infected D17 cells with an efficiency of about 10⁵ (=100,000) colony forming units per ml of tissue culture supernatant medium. Such virus particles also infected D17 cells conjugated with DNP. However, the efficiency of infection was three orders of magnitude less than that of cells not conjugated with DNP. This drop in virus titer is mainly due to difficulties of selecting DNP conjugated cells with the antibiotic. It appears that the conjugation reaction makes cells very vulnerable to the drug and more than 90% of the cells died two to three days after the conjugation action. Virus particles with wild-type envelope do not infect CHO cells.

The mutation of the cleavage site of the envelope precursor protein (SNV-env-mD) completely abolished infectivity. Only one colony was observed in D17 cells not conjugated with DNP. This finding coincides with earlier reports that mutations in the envelope precursor cleavage site lead to non-infectious virus particles. Cells transfected with pTC4 (Figure 2) did not produce vector particles that were able to infect D17 or CHO cells at significant efficiencies. Cells transfected with pTC5 produced virus particles unable to infect D17 or CHO cells. However, such particles significantly infected cells conjugated with DNP.

### Wild-Type Envelope

First, the presence of wild-type envelope in particles displaying an antigen binding site against DNP was tested to determine whether there would be an increase in the efficiency of infection of cells conjugated with DNP. It was found that DNP conjugated HeLa cells could not be infected with vector virus particles that contained the wild type envelope alone. However, DNP conjugated cells could be infected with anti-DNP displaying retroviral vectors at a very low efficiency. The titer measured was about 10 infectious units per ml of tissue culture supernatant medium. Virus particles that contained wild-type envelope in addition to the targeting anti-DNP envelope infected cells 10 to 30 times more efficiently. This data indicate that the presence of wild-type envelope can increase the efficiency of infection of targeting vectors. Two additional sets of experiments using other targeting molecules were performed to corroborate this finding.
1. Infectivity studies with virus particles containing antibody-envelope fusion proteins. D17 cells, HeLa cells and Col-1 cells were infected with virus particles displaying an antigen binding site of an antibody (B6.2, Bird, R.E. et al., 1988 and Colcher, D. et al., 1990) directed against a cell surface protein expressed on various human carcinoma cells. Vector virus particles were harvested from a variety of different helper cell lines (Table 2). All virus particles were carrying a vector transducing the bacterial beta-galactosidase gene. Infectivity was determined by staining the cells with X-gal as described (Mikawa, T. et al.). The number of blue cell colonies was determined two to three days after infection. The following virus particles were tested for infectivity: virus particles that do not contain envelope (termed "no env"), virus particles that contain wild-type envelope alone (termed wt-env - DSN), virus particles that contain targeting envelopes alone which are antibody-envelope fusion proteins (termed TC24, TC25, and TC26 as described in Figure 6), and particles that contain wild-type plus targeting envelopes (termed TC24+wt-env, TC25+wt-env, and TC26+wt-env).
   Particles that do not contain any envelope were found to be basically not infectious. Particles that contain wild-type envelope were infectious only on D17 cells which contain a viable receptor for wild-type SNV. The particles were not infectious on HeLa cells or Col-1 cells. Particles that contained targeting envelopes only were infections on D17 and HeLa cells. The efficiency of infection on D17 cells was less than 5% of that of virus containing wild-type envelope. Such particles were not infectious on Col-1 cells. The addition of wild-type envelope increased efficiency of infection 10 to 50 fold. Further, Col-1 cells that could not be infected with particles containing either envelope alone could be infected with particles containing both wild-type env and targeting env. This data indicates that the wild-type envelope adds a function improving or even completely enabling virus penetration (Table 2). These data also show that the level of infectivity is dependent on the position within the envelope gene at which the antibody is fused to the envelope.
2. Infectivity studies with virus particles containing SNV-MLV-fusion proteins. CHTG cells (described in Albritton, L.M. et al., 1989) expressing the receptor of ecotropic murine leukemia virus as well as D17 cells were infected with virus harvested from cells expressing targeting envelopes (SNV-MLV-chi-C and SNVMLV-chic-D) alone or from helper cells expressing targeting envelope plus wild-type envelope. Virus particles were carrying to hygromycin B resistance gene. Infected cells were selected from hygromycin resistance and the number of hygromycin resistant cell colonies was determined. Retroviral vector particles containing the targeting envelope alone were not infectious. The particles became infectious after wild-type envelope was added to the particles. Particles with wild-type envelope alone are not infectious on CHTG cells (Table 3).

### Discussion

### Targeting Envelope

The data obtained with retroviral particles containing antibody-envelope fusion proteins showed that such particles are competent for infection. Surprisingly, TC4, a construct that contains the scFv gene fused to *env* in the middle of SU did not give virus particles capable of binding DNP. This may be due to an unstable SU-TM complex. This hypothesis is supported by the finding that such particles failed to bind to DNP-BSA-Sepharose. Low level infectivity of such particles on D17 cells may result from unspecific adsorption of virus particles containing TM only. Unspecifically adsorbed virus particles (depleted of SU) may occasional penetrate the cell.

Cells transfected with pTC5 produce virus particles with chimeric envelopes without a functional retroviral membrane fusion domain. This assumption is based on the finding that virus particles containing uncleaved envelope precursor proteins (SNV-env-mD) are not infectious. However, it is known that some antibody molecules are internalized by cells after binding to cell surface by an unknow mechanism. The data show that such an internalization mechanism might be sufficient to allow internalization of the virus particle and the consequent establishment of a successful infection.

### Applications of Vector Particles With Antibody-Envelope Fusion Proteins in Gene Therapy

In all applications of human gene therapy so far, the cells of interest were isolated from the patient, purified from other cell types, and infected in tissue culture with retroviral vector particles which were harvested from helper cells. After expansion of the treated cells in tissue culture, they were re-injected into the patient. The infection of cells has to be done *in vitro,* since the retroviral vector particles used (derived from amphotropic murine retroviruses) have a broad host range. Thus, if injected directly into the blood stream of a patient, such virus particles would infect all kinds of tissue. Besides other risks, this treatment would be inefficient, since the chance that the gene will be delivered to its appropriate target cell is very low.

This clinical gene therapy protocol may be sufficient to obtain insight into how efficient and how beneficiary gene therapy will be for the patient. Indeed, the clinical data look very promising (Eglitis, personal communication). However, the current clinical protocol is very laborious, time consuming, very costly, and, therefore, not suitable for general clinical application. For general clinical application, it will be necessary to inject the gene transfer vehicle directly into the body of the patient.

The development of a retroviral vector particle that only infects one specific cell type, may allow the direct injection of the vector into the patient's blood stream. The development of vector particles containing antibody-envelope chimeras may be the first step towards this goal and may open a new area of possible applications of gene therapy *in vivo*.

### In Vivo Delivery Of Therapeutic Genes With Retroviral Vectors That Display Single Chain Antibodies

To test in vivo gene delivery with retroviral vector particles that display single chain antibodies on the viral surface. SCID mice have been used as a first model system. It is well known that retroviral vectors are rapidly inactivated by complement, if they are produced from cells of a heterologous species. However, SCID mice do not have a functional immune system.

### Confirmation that SCID mouse serum does not inactivate SNV retroviral particles.

In our experiments, retroviral vector particles were harvested from DSH-cxl packaging lines (described in Martinez and Dornburg. Virology, 208: 234-241, 1995; Chu and Dornburg, J. Virol. 69: 2659-2663, 1995). These packaging cells transduce a retroviral vector expressing the bacterial β-galactosidase gene (*lacZ* gene). The plasmid pCXL (Mikawa T.D., et al., Exp. Cell Res., 195: 516-523, 1992) contains a SNV-derived retroviral vector expressing the *lacZ* gene. Aliquots of 100 µl of the retroviral vector solution were prepared and incubated with 0.1, 1, 10, and 100 µl of SCID mouse serum for 10 minutes, respectively. In a second experiment, the same amounts of heat-inactivated (60°C for 30 minutes) SCID mouse serum were added to 100 µl of vector stocks. Next, the mixtures were added to 2 x 10⁵ D17 cells in infectivity assays, Two days after this infection the cells were stained with x-gal and the number of blue colonies was determined as described (Martinez and Dornburg, Virology, 208: 234-241, 1995: Chu and Dornburg, J. Virol 69: 2659-2663, 1993). No significant differences in infectivity were observed between samples incubated with fresh or heat-inactivated serum. Thus, the conclusion can be made that SCID mouse serum does not contain antibodies and/or complement factors which inactivate SNV retroviral particles.

**Cell type specific gene delivery** ***in vivo.*** In another experiment, hygromycin-resistant human COLO320DM cells, which express the cell surface marker Her2neu. (ATCC accession No. CCL 220) or hygromycin-resistant A431 cells, which do not express Her2neu (ATCC accession No, HB-9629), were injected into the peritoneum of mice. The next day, vector virus particles, which displayed anti-Her2neu single chain antibodies (10⁶ infectious particles) were injected. The preparation of these virus particles is described below. The vector virus particles transduced a marker gene, the bacterial beta-galactosidase gene, into infected cells. Thus, by staining cells after infection, gene transduced cells stain blue and, therefore, can be easily detected.

**Construction of packaging cell lines.** The construction of stable packaging cells, which produce retroviral vector particles displaying the anti-Her2neu antibody followed the protocol described in detail in Chu. T. -H. and Dornburg. R., J. Virol 71: 720-725, 1997. Briefly, using the dog D17 cell-derived cell line DSgp13-cxl which expresses the encapsidation-negative SNV gag-pol proteins and the packagable retroviral vector pCXL, cell lines were made which expressed the chimeric scA-env fusion proteins. For this, the scA-Env gene expression vector pAJ7 (described below) was transfected into DSgp13-xcl cells in co-transfection with a plasmid expressing a selectable marker gene. In this experiment, we used the puromycin resistance gene, driven by the SNV promoter, contained on pRD118-puro, derived from pRD118 (Chu, T. H. et al., Bioteching, 18: 890-899, 1995). However, one skilled in the art would be able to readily to select an appropriate selectable marker gene. About 100 to 200 single antibiotic resistant cell colonies were isolated for each transfection. Expression of the scA-Env protein was tested by ELISA assays and infectivity assays as soon as cells had been transferred to 24 well plates. The reason for making helper cells this way is that transfected plasmid DNAs often integrate into rather inactive chromosomal sites are poorly transcribed.

Next, the SNV wild-type envelope gene expression vector pIM29 (Martinez. I. and Dornburg. R., J. Virol, 69:4339-4346, 1995: and Martinez. I. and Dornburg. R. Virology 208:234-241, 1995) was transfected into cell lines established from the single colonies described above. Again, the transfection was done by co-transfecting a plasmid expressing a selectable marker (e.g., the hygromycin B phosphotransferase gene). 100 to 200 single cell colonies were isolated and tested for infectivity on human target cells. Cell clones with the highest infectivity were selected, re-cloned once or twice, and finally used for in vivo experiments.

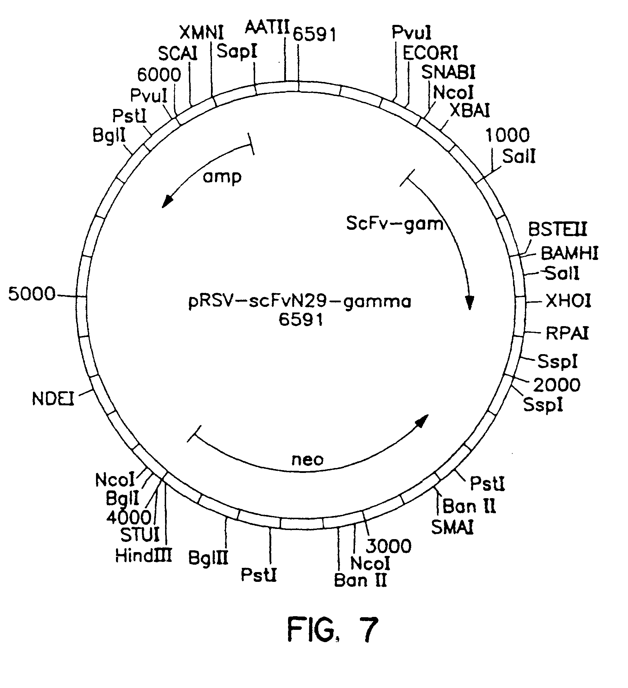

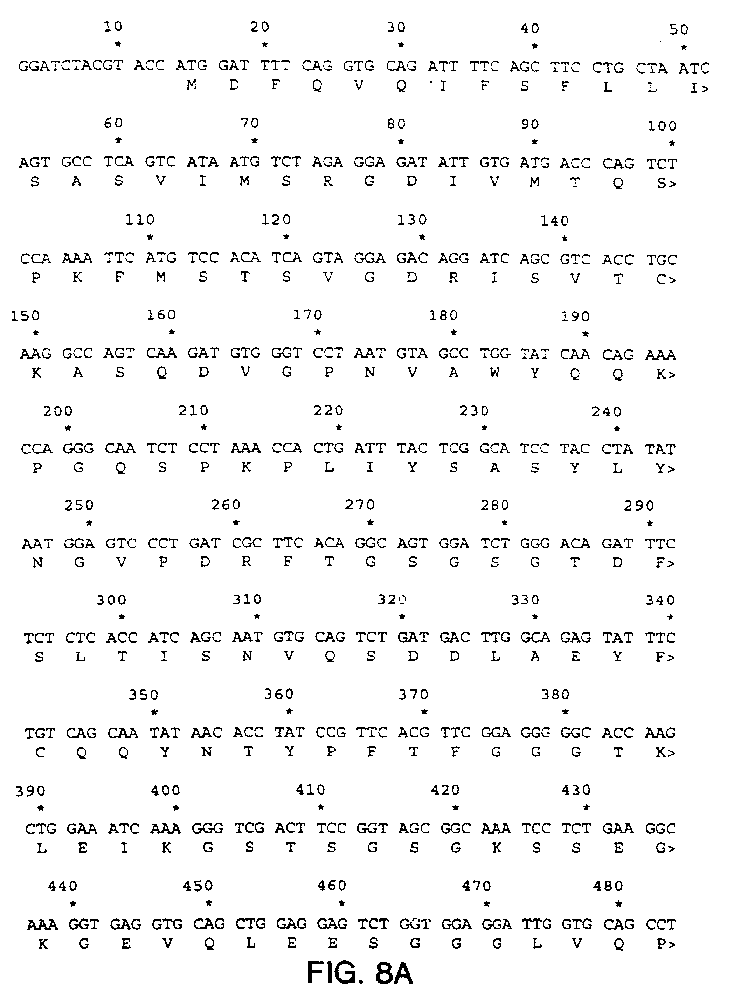

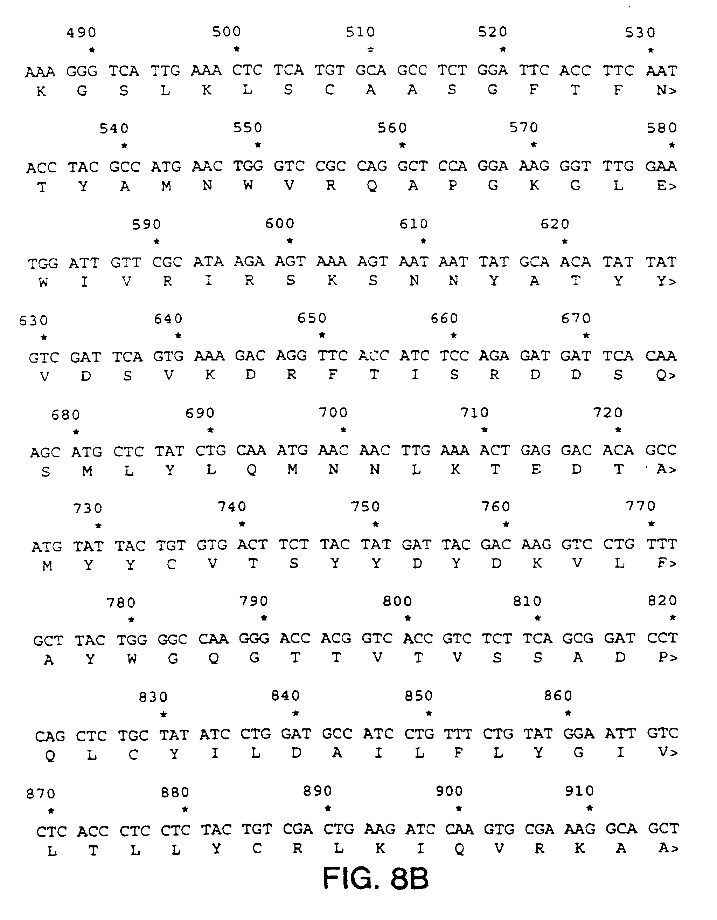

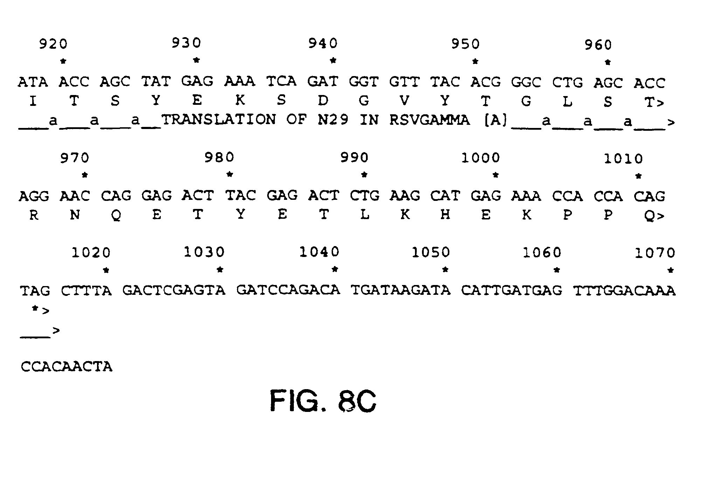

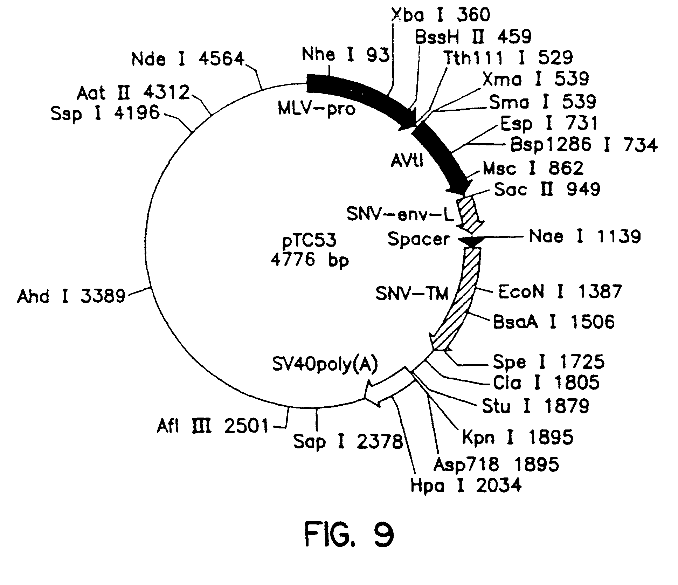

pAJ7 is a plasmid containing the anti-Her1neu scA fused to the SNV-Env-TM coding region. This plasmid is very similar to plasmid pTC25 (Figure 6). However, it was made in a slightly different way: First, referring to FIGs. 7 and 8, a DNA linker coding for the amino acids ala-gly-ala-ser-gly-ser was inserted at the carboxy terminal end of the anti-Her2neu scA gene (which contains the authentic hydrophobic leader sequence of the antibody-gene for transport through the ER) in plasmid N29-gamma to give plasmid pRD161 (not shown). A DNA fragment (SnaB1 to Eco47111) isolated from pRD161 and which contained the complete anti-Her2neu scA (including the hydrophobic leader sequence) were cloned into pTC53 (FIG. 9) digested with Sac2 (blunt ended) plus Nael to give plasmid pAJ7. DNA sequencing was performed after all cloning steps to verity the maintenance of the correct reading frame of genes coding for the chimeric protein.

Plasmid pTC53 (FIG. 9) is a gene expression plasmid for the fast cloning and efficient expression of single chain antibody-envelope fusion proteins for display on retroviral particles. pTC53 has been derived from pTC13 (Chu, T.-H. and R. Dornburg, 1995. J. Virol. 69: 2659-2663; and Chu, T.-H., et al., 1995, Biotechniq. 18: 890-899). It contains the MLV-U3 promoter and the Adenovirus 2 tripartite leader sequence followed by a sequence coding for the hydrophobic leader sequence of the SNV envelope gene. Next, the plasmid contains a unique NaeI site (which cuts between two codons) for cloning of single chain antibody genes (e.g. PCR products). Downstream of the NaeI site is a DNA linker coding for the amino acid motif (gly₄-ser)₃ to enable flexibility of the scA. This gly-ser linker is fused in frame to the complete transmembrane peptide (TM) coding region of the SNV envelope gene. The polyadenylation site downstream of the TM coding region has been derived from simian virus 40 (SV40). The plasmid backbone is that of pUC19.

**Concentration of vector virus stocks.** Concentrated vector virus stocks were prepared as described by Chu and Dornburg. J. Virol, 71:720-725, 1997. Briefly, to concentrate the virus particles by ultratiltration, 15 ml of supernatant was added to Amicon Centriprep filtration devices containing 100, or 500 kD molecular weight cut-off (MWCO) membranes (Amicon, Beverly, MA). The 100 kD cut-off membranes are made of cellulose, the 500 kD membrane is made of polysulfone (David Brewster, Amicon, personal communication). The vector particle solution was centrifugated in a Sorvall RC5000B table top centrifuge with swing-out buckets at 3,000 rpm for 30 min at 20°C. The supernatant medium was discarded and the solution was centrifugated at least one more time for 10 minutes or until a final volume of 1 ml for less, e.g., 0.7 ml) was obtained. The final concentrate was used immediately for the infectivity experiments.

**Confirmation of cell-type specific gene delivery** ***in-vivo.*** The day after vector virus injection, the mice were sacrificed. Human cells were recovered by trypsin treatment of the pentoneum. The cells were isolated and subjected to different antibiotic selection in tissue culture. Hygromycin selection enables the growth of human COLO320DM Her2neu+ target cells or A431 Her2neu- non-target cells but not the mouse cells. In both experiments, human cells started to grow as colonies in a vast background of mouse cells. 4 days after selection, the cells were stained with x-gal. (Chu, T. -H. and R. Dornburg. 1997, J. Virol 71: 720-725). We found that 2 to 3% of the COLO320DM target cells were infected *in vivo*. None of the A431 were infected. Furthermore, none of the mouse cells (mainly epithelial cells of the peritoneum) stained blue indicating that they were not infected. This data indicates that a cell-type specific gene delivery can be obtained *in vivo* and that SCID mice are suitable model systems to study *in vivo* gene delivery.

### Wild-Type Envelope

Retroviral vector particles which display an antigen binding site of an antibody can specifically infect cells that contain an antigen specific for the antibody. However, the efficiency of the gene transfer can be low. We hypothesized that the fusion of the targeting peptide to the envelope impaired the natural fusion function of the envelope which is essential for efficient penetration of the virus. Thus, the hypothesis that the addition of a wild-type envelope may complement this shortcoming was tested.

New retroviral vector particles containing two different types of targeting envelopes were constructed. These targeting envelopes were (1) fusion proteins containing the antigen binding site of an antibody fused to various carboxy terminal portions of the envelope protein of spleen necrosis virus, SNV; and (2) fusion proteins consisting of the receptor binding domain of ecoMLV fused to various carboxy terminal portions of the SNV, similar to the antibody envelope constructs (Figure 6).

Targeting envelopes alone are little or not infectious on cells that contain a receptor for the targeting envelope. The addition of wild-type envelope to particles containing targeting envelopes dramatically increased or even completely enable infectivity on target cells that could hardly or not at all infected with virus particles containing either envelope alone. This data show that the construction of particles containing mixed envelopes dramatically improves the efficiency of gene transfer into specific target cells and, therefore, provides a valuable tool to introduce genes into specific target cells.

This method can be probably be improved by mutating the natural receptor binding domain of the wild-type envelope (e.g.. by site directed mutagenesis). Using a wild-type envelope containing a non-functional receptor binding site in mixed envelope retroviral vector particles may enable to also target cells that contain a receptor for the wild-type envelope without loosing target cell specificity.

**Table 1**

| **Infectivity of Retroviral Vector Particles On D17 and CHO Cells With and Without DNP Conjugation*** | | | | | |
|---|---|---|---|---|---|
| **Virus titer (cfu/ml)** | | | | | |
| **Envelope of virus particle** | **Binding to DNP** | **D17 cells** | **D17+DNP** | **CHO-DNP** | **CHO+DNP** |
| SNV-env-mC | nd | 10⁵ | 10² | 0 | 0 |
| SNV-env-mD | -- | 1 | 0 | 0 | 0 |
| TC4 | -- | 10 | 0 | 0 | 1 |
| TC5 | + | 0 | 10² | 0 | 10 |

| | | | | | |
|---|---|---|---|---|---|
| * Virus was harvested from tissue culture cells expressing SNV gag-pol and the envelope protein indicated n the left column (see also Figure 2). All cells contained pJD214HY, a retroviral vector expressing the hygromycin B phosphotransferase gene. Infected cells were selected for hygromycin resistance. The number of hygromycin resistant cell colonies was determined two to three weeks after infection (after all cells had died in uninfected control plates). DNP binding of vector particles was the determined by measuring reverse transcriptase activity bound to DNP-BSA-Sepharose particles. nd: not determined; 0: no hygromycin resistant colonies were detected. Virus titers are expressed as colony forming units (cfu) per ml of tissue culture supernatant medium. | | | | | |

**Table 2**

| **Infectivity of Retroviral Vector particles Displaying the B6.2 Single Chain Antigen Binding Peptide** | | | |
|---|---|---|---|
| **Construct** | **Titer(CFU/ml)** | | |
| **gag-pol +** | **D17** | **HeLa** | **COL-1** |
| no env | 1 | nd | -* |
| wt-env (dsn) | 1.000 | nd | nd |
| TC26 | 40 | 10 | nd |
| TC24 | 20 | 5 | nd |
| TC25 | 45 | 20 | nd |
| TC26 - wt-env | 1.100 | 90 | 45 |
| TC24 - wt-env | 10.000 | 250 | 115 |
| TC25 - wt-env | 4.000 | 100 | 55 |
| Plasmid constructs pTC24, pTC25 and pTC26 were transfected into D17 cells that express retroviral core proteins (gag-pol) and the vector pCXL (Mikawa, T.), or into the retroviral packaging line DSN also containing the pCXL vector (in co-transfection with a plasmid expressing an antibiotic resistance gene). The pCXL vector transfers the bacterial. B-galactosidase (taco gene. Virus was harvested from stable transfected cell-lines and fresh D17 cells. HeLa cells, or Col-1 cells were infected. Two days after infection, cells were stained with X-gal. Blue cells indicate infected cells expressing the lacZgene. Virus titers are expressed as colony forming units per ml tissue culture supernatant medium harvested from helper cells (cful ml). | | | |

| | | | |
|---|---|---|---|
| -* experiment not done. Nd: no infected cells were detected in infection experiments using a total of 2 ml supernatant tissue culture medium. | | | |

**Table 3**

| **lnfectivity of Retroviral Vector Particles Containing Chimeric Envelope Proteins of MLV and SNV** | | |
|---|---|---|
| | **Virus titer (cfu/ml)** | |
| **Envelope of virus particle cells** | **D17 cells** | **CHTG** |
| MLY-SNV-chiC | nd | nd |
| MLV-SNV-chiD | nd | nd |
| MLV-SNV-chiC - wt SNV | 10⁶ | 10³ |
| MLV-SNV-chiD - wt SNV | 10⁶ | 10³ |
| DSN | 10⁵ | nd |
| Virus was harvested from tissue culture cells expressing SNV gag-pol and the envelope protein indicated in the left column (see also Figure 6). All experiments were performed with pJD214HY a retroviral vector transferring the hygromycin resistance gene. Virus titers are expressed as hygromycin resistant colony forming units per ml of tissue culture supernatant medium. SNV-MLV-chiC+wt SNV and SNV-MLV-chiD+wt SNV are cell lines expressing chimeric envelopes of MLV and SNV plus the envelope of wild type (wt) SNV. DSN cells are SNV based helper cells expressing gag-pol and SNV env from two different plasmid constructs. Virus titers are expressed as colony forming units (cfu) per ml of tissue culture supernatant medium. Nd: no hygromycin resistant colonies were detected using a total of 5ml tissue culture medium. | | |

The term "oligonucleotide" as used herein refers to primers, probes, oligomer fragments to be detected, oligomer controls, and unlabeled blocking oligomers. Oligonucleotide are molecules comprised of two or more deoxyribonucleotides or ribonucleotides. The term "primer" as used herein refers to an oligonucleotide, preferably an oligodeoxyribonucleotide, either naturally occurring such as a purified restriction digest or synthetically produced, which is capable of acting as a point of initiation of synthesis when subjected to conditions in which synthesis of a primer extension product, which is complementary to a nucleic acid strand, is induced. *i.e.,* in the presence of nucleotides, an agent for polymerization such as a DNA polymerase, and a suitable temperature and pH. The primer must be sufficiently long to prime the synthesis of extension products in the presence of the polymerization agent. Methods for amplifying and detecting nucleic acid sequences by polymerase chain reaction (PCR) are described in detail in United States patents no. 4,683,195, 4,683,202, and 4,965,288.

Figure 1 is a diagram illustrating helper cells expressing retroviral proteins. A) Helper cells are made by the transfection of plasmids expressing all retroviral proteins necessary to form infectious virus particles. One plasmid is designed to express all core/proteins (expression of gag and pol). The other plasmid is designed to express the envelope precursor-protein. Both plasmid constructs do not contain retroviral cis/acting sequences for virus replication (e.g.. encapsidation sequences, a primer building site etc.).. Polyadenylation takes place in non retroviral polyadenylation recognition sequences. B) After transfection of the retroviral vector, the vector RNA genome is encapsidated into core structures. The helper cell is producing retroviral particles that only contain the vector genome with the gene(s) of interest. The vector contains all cis/acting sequences for replication. Thus, in infected target cells, the vector genome is reverse transcribed and integrated into the genome. Due to the lack of retroviral protein coding genes in the vector genome, no virus particles are produced from infected target cells. C) Helper cells that contain a plasmid express a modified envelope gene. The helper cell is very similar to that shown above. However, chimeric envelope genes were constructed that contain the antigen binding domain of an antibody at the amino terminus fused to the carboxy terminus of the envelope gene. Such particles may only bind to and infect target cells that contain an antigen structure which is recognized by the antibody moiety of the chimeric envelope protein.

Figure 2 is a diagram illustrating plasmids expressing mutant envelope genes of spleen necrosis virus (SNV). Genes are expressed from the Rous sarcoma virus promoter (RSV pro) and polyadenylated within the polyadenylation signal of herpes simplex virus thymidine kinase gene (TK poly(A)). The polylinker of pBluescript was inserted between the promoter and the polyadenylation sequence to allow the easy cloning of genes into this vector (plasmid sequences that abut the vector are not shown). a/b) point mutations were introduced into the *env* gene by site directed mutagenesis to create new restriction enzyme recognition sites (indicated by an *). All enzymes cut exactly between two codons creating blunt ends for easy ligation without shifting the reading frame, c/d) chimeric envelope of containing an antigen binding peptide fused to the carboxy terminus of env. e) pJD214Hy, a retroviral vector used in all studies to test the transfer of genes by retroviral vector particles.

Figure 3 shows the sequence of the single chain antibody gene (scFv) against the hapten DNP.

Figure 4 illustrates retroviral packaging cells. A) A eucaryotic cell containing two different plasmids for the production of retroviral vector particle proteins. A retroviral vector transfected into such cells and carrying the gene of interest is encapsidated by retroviral core proteins. The envelope expression vectors expresses targeting envelopes (e.g., an antigen binding peptide fused to the envelope). Virus particles are produced that infect a target cell only that contains a receptor specific for the antigen binding site. The helper shown under B) is similar to that shown above except that it also contains a gene expression vector coding for the wild-type envelope. Virus particles produced from such helper cells contain "mixed" envelopes which consist of the targeting envelope and the wild-type envelope. Formation of mixed oligomers is possible because both, the targeting as well as the wild-type envelope contain a complete TM peptide which mediates the formation of oligomers.

Figure 5 illustrates a eucaryotic gene expression vector (pTC13) to obtain high level of gene products that contain an ER recognition sequence. The vector shown has been derived from another gene expression vector (termed pRD114) which is described in Sheay. W. et al., 1993. The vector shown differs from pRD114 in that it contains a gene fragment coding for an ER recognition signal sequence to enable the transport of proteins through the endoplasmatic reticulum. It contains two recognition sites for the restriction enzymes Nrul and Stul which cut between two codons downstream of the ER signal sequence coding region. DNA fragments coding for any peptide can be inserted into this vector Translation of the inserted gene is terminated by using one of the three stop codons. MLV-U3-pro: promoter and enhancer of murine leukemia virus: Ad V. leader: impartite leader sequence of adenovirus: SV40 poly (A): polyadenylation signal sequence of simian virus 40.

Figure 6 illustrates plasmid sectors expressing targeting envelope proteins. The PCR product of the gene coding for the single chain antibody B6.2 (a Eco4111 to Nrul fragment, see Material and Methods) was cloned into the Nrul site of pTC13 (Figure 5) to give plasmid pTC23. Carboxy terminal parts of the SNV envelope gene were isolated and cloned into the NruI site downstream of the B6.2 antibody gene. The resulting targeting antibody-envelope fusion gene of pTC24 and pTC25 are similar to pTC4 and pTC5. pTC24 and pTC25 retain exactly the same amount of SNV envelope as pTC4 and pTC5, respectively. In pTC26 the antibody coding genes abuts the envelope coding region at codon 167 of the envelope gene. Plasmids PSNV-MLV-chiC and pSNV-MLV-chiD are identical to pTC4 and pTC5, except that the antibody gene is replaced with a gene fragment encoding for almost the complete SU peptide of MLV. In these clones the ER transport signal sequence (L) is from MLV. MLV-pro: promoter and enhancer of murine leukemia virus: Avtl: tripartite leader sequence of adenovirus: L: ER transport signal sequence. B6.2scFV: gene encoding the single chain antibody B6.2: poly(A) polyadeylation signal sequence of SV40: SU surface peptide coding region of the SNV envelope: TM: transmembrane coding region of the SNV envelope: RSV: promoter and enhancer of Rous sarcoma virus.

### Appendium of References

Albritton, L.M., Tseng, L., Scadden, D., and Cunningham, J.M. 1989. A putative murine ecotropic retrovirus receptor gene encodes a multiple membrane-spanning protein and confers susceptibility to virus infection. Cell 57:659-666.
Battini, J.L., Heard, J.M. and Danos, O. (1992) Receptor choice in the envelope glycoproteins of amphotropic, xenotropic, and polytropic murine leukemia viruses. J. Virol. 66:1468-1475.
Bird, R.E., Hardman, K.D., Jacobson, J.W., Johnson, S., Kaufman, B.M., Lee, S.M., Lee, T., Pope, S.H., Riordan, G.S., and Whitlow, M. 1988. Single-chain antigen binding proteins. Science, 242: 423-426.
Chu, T. -H. and R. Dornburg, 1995. Retroviral vector particles displaying the antigen-binding site of an antibody enable cell-type-specific gene transfer. J. Virol. 69: 2659-2663.
Chu, T. -H. and R. Dornburg, 1997. Towards highly-efficient cell-type-specific gene transfer with retroviral vectors that display a single chain antibody. J. Virol. 71: 720-725.
Chu, T. -H.. I. Martinez, P. Olson, and R. Dornburg, 1995. Highly efficient eucaryotic gene expression vectors for peptide secretion. Biotechniq. 18: 890-899.
Colcher, D., Bird., R., Roselli, M., Hardman, K.D., Johnson, S., Pope, S., Dodd, S.W., Pantoliano, M.W., Milenic, D.E., Schlom, J. 1990. *In vitro* tumor targeting of a recombinant single chain antigen-binding protein. J. Natl. Canc. Inst. 82: 1191-1197.
Cone, R.D. and Mulligan, R.C. 1984. High-efficiency gene transfer into mammalian cells: generation of helper-free recombinant retrovirus with broad mammalian host range. Proc. Natl. Acad. Sci. USA 81: 6349-6353.
Cournoyer, D., Scarpa, M., Jones, S.N., Moore, K.A., Belmont, J.W., and Caskey, C.T. 1990. Gene therapy: a new approach for the treatment of genetic disorders. Clin. Pharmacol. Ther. 47(1): 1-11.
Danos, O. and Mulligan, R.G. 1988. Safe and efficient generation of recombinant retroviruses with amphotropic and ecotropic host ranges Proc. Natl. Acad. Sci. 85: 6460-6464.
Davis, M.M. and Metzger, H. 1983. Structural basis of antibody function. Annu. Rev. Immunol. 1:87 117.
Dougherty, J.P. and Ternin, H.M. 1989. New retrovirus helper cells with almost no nucleotide sequence homology to retrovirus vectors. J. Virol. 63. 3209-3212.
Friedman, T. 1989. Progress toward human gene therapy. Science 244, 1275-1281.
Gritz, L., and J. Davies 1983. Plasmid encoded hygromycin B resistance: the sequence of hygromycin B phosphotransferase gene and its expression in Escherichia coli and Saccharomyces cerevisiae. Gene 25, 179-188.
Hunter, E. and Swanstrom R. 1990. Retrovirus envelope glycoproteins. Curr. Top. Microbiol. Immunol. 157:187-253.
Kohn, D.B., Anderson, W.F., and Blaese, M.B., 1989. Gene therapy for genetic diseases. Cancer Investigation 7(2):179-192.
Larson, S.M. 1990. Improved tumor targeting with radiolabeled, recombinant, single-chain, antigen-binding protein. J. Natl. Canc. Inst. 82: 1189-1190.
Markowitz, D., Goff, S., and Bank, A. 1988. A safe packaging line for gene transfer: separation of viral genes on two different plasmids. J. Virol. 62:1120-1124.
Martinez, I. and R. Dornburg, 1995. Mapping of receptor binding domains in the envelope protein of spleen necrosis virus. J. Virol. 69: 4339-4346.
Martinez, I. and R. Dornburg, 1995. Improved retroviral packaging lines derived from spleen necrosis virus. Virology 208: 234-241.
Mikawa, T., Fischman. D.A., Dougherty, J.P. and Brown, A.M.C. In vivo analysis of a new lacZ retrovirus vector suitable for lineaee marking in avian and other species. Exp. Cell Res. 195: 516-523.
Miller, A.D. 1990. Progress toward human gene therapy. Blood 76(2) 271-278.
Mims, C.A. 1986. Virus receptors and cell tropism. J. Infect. 12:199-204.
Ott, D., and Rein, A. 1992. Basis for receptor specificity of nonecoptropic murine leukemia virus surface glycoprotein gp70SU. J. Virol. 66:4632-4638.
Riley, S.C., Connors. S.J., Klinman, N.R., and Ogata, R.T. 1986. Preferential expression of variable region heavy chain gene segments by predominant 2,4-dinitrophenyl-specific BALB'c neonatal antibody clonotypes. Proc. Natl. Acad. Sci. 83:2589-2593. Rolt. L. 1988. In "Essential Immunology". Blackwell Scientific Publications.
Sheay, W., Nelson, S., Martinez, I., Chu, T.H.T., Bathia, S. and Dornburg, R. 1993. Dowstream insertion of the adenovirus tripartite leader sequence enhances expression in universal eucaryotic vectors. Biotechniques. 15:856-861.
Shinnick, T.M., Lerner, R.A. ad Sutcliffe, J.G. 1981. Nucleotide sequence of Moloney murine leukemia virus. Nature 293:543-548.
Ternin, H.M. 1986. Retrovirus vectors for gene transfer: efficient integration into and expression of exogenous DNA in vertebrate cell genomes, in "Gene Transfer" (R. Kucherlapatl. ed.) Plenum Press, New York.
Varmus. H.E. and Brown. P. 1988 Retroviruses, in "Mobile DNA" (M. Howe and D. Berg, eds.) ASM, Washington, DC.
Watanabe, S. and Ternin, H.M. 1983. Construction of a helper cell line for avian reticulonendotheliosis virus cloning vectors. Mol. Cell. Biol. 3.2241-2249.
Weiss, R., Teich, N., Varmus, H. and Coffin, J. 1985. RNA tumor viruses. Cold Spring Harbor Laboratory, Cold Spring Harbor, New York.
Whitlow, M. and Filpula, D., Single-chain Fv proteins and their fusion proteins. Methods: A comparison to methods in Enzymology. Vol. 2 pp. 1-9.

## Claims

1. Use of a retroviral vector having an envelope protein wherein a viral receptor portion is at least partially defined by a single chain antibody and said viral receptor portion binds to a selected antigen in the manufacture of a medicament for use in infecting in vivo a cell having the said selected antigen wherein the said medicament is for contacting said retroviral vector in vivo with the cell such that said viral particle or a portion thereof is internalized into the cell, and the cell having the selected antigen is infected by said retroviral vector.

2. The use of Claim 1 wherein said retroviral vector is produced using a plasmid comprising a nucleotide sequence encoding a single molecule comprising a portion of an envelope protein and a portion of a single chain antibody.

3. The use of Claim 2 wherein said nucleotide sequence encodes a portion of the envelope protein of Spleen Necrosis Virus.

4. The use of Claim 2 wherein the plasmid is that identified in Figure 2d as pTC5.

5. Use of a retroviral vector having a targeting peptide fused to the envelope protein of said retroviral vector to form a targeting envelop in the manufacture of a medicament for infecting in vivo a cell having a selected antigen wherein the said medicament is for contacting said retroviral vector in vivo with the cell such that said viral particle or a portion thereof is internalized into the cell, and the cell having the selected antigen is infected by said retroviral vector.

6. The use according to Claim 5 wherein said targeting peptide replaces or disrupts the natural viral receptor binding site.

7. The use of Claim 5 wherein said targeting peptide is the antigen binding site of an antibody.

8. The use of Claim 5 wherein said targeting peptide is a peptide that specifically binds to a specific receptor of the target.

9. Use of a retroviral vector particle having target cell specificity which comprises a retroviral vector having an antigen binding site of an antibody fused to the envelope protein of the retroviral vector, wherein the antigen binding site of the antibody replaces the natural viral receptor binding site, in the manufacture of a medicament for a cell type specific method for introducing in vivo genes into vertebrate cells using retroviral vectors.

10. The use of Claim 5 or 9 wherein the retroviral particle is spleen necrosis virus.

11. The use of Claim 5 or 9 wherein the targeting peptide or antibody is a single chain antibody against the hapten dinitrophenol.

12. The use of Claim 5 or 9 wherein the targeting peptide is an antigen binding site directed against a cell-surface protein of the target cell.

13. The use of Claim 5 or 9 wherein the targeting peptide is the receptor binding peptide of another virus.

14. The use of Claim 5 or 9 wherein the retroviral vector comprises a targeting envelope and a wild-type envelope.

15. The use of Claim 14 when dependent on Claim 9 wherein the wild type envelope is derived from spleen necrosis virus.

16. The use of claim 9, or of claim 10, 12, 14 or 15 when dependent on claim 9, wherein the antibody is an anti-Her2neu single chain antibody.

## Patentansprüche

1. Verwendung eines Retrovirusvektors mit einem Hüllprotein, wobei ein Virusrezeptorabschnitt zumindest teilweise von einem einkettigen Antikörper gebildet ist, und sich der Virusrezeptorabschnitt an ein ausgewähltes Antigen bindet, bei der Herstellung eines Medikaments, das dazu verwendet wird, eine Zelle mit dem ausgewählten Antigen in vivo zu infizieren, wobei das Medikament den Retrovirusvektor *in vivo* so mit der Zelle in Kontakt bringen soll, dass das Viruspartikel oder ein Teil davon in die Zelle aufgenommen und die Zelle mit dem ausgewählten Antigen vom Retrovirusvektor infiziert wird.

2. Verwendung nach Anspruch 1, wobei der Retrovirusvektor unter Verwendung eines Plasmids hergestellt wird, das eine Nucleotidsequenz umfasst, die ein Einzelmolekül codiert, das einen Teil eines Hüllproteins und einen Teil des einkettigen Antikörpers umfasst.

3. Verwendung nach Anspruch 2, wobei die Nucleotidsequenz einen Teil des Hüllproteins des Milznekrosevirus codiert.

4. Verwendung nach Anspruch 2, wobei das Plasmid dasjenige ist, das in Figur 2d als pTC5 **gekennzeichnet** ist.

5. Verwendung eines Retrovirusvektors mit einem Empfängerpeptid, das am Hüllprotein des Retrovirusvektors fixiert ist, um eine Empfängerhülle zu bilden, bei der Herstellung eines Medikaments, das dazu verwendet wird, eine Zelle mit einem ausgewählten Antigen in vivo zu infizieren, wobei das Medikament den Retrovirusvektor in vivo so mit der Zelle in Kontakt bringen soll, dass das Viruspartikel oder ein Teil davon in die Zelle aufgenommen und die Zelle mit dem ausgewählten Antigen vom Retrovirusvektor infiziert wird.

6. Verwendung nach Anspruch 5, wobei das Empfängerpeptid die natürliche Virusrezeptorbindestelle ersetzt oder unterbricht.

7. Verwendung nach Anspruch 5, wobei das Empfängerpeptid die Antigenbindestelle eines Antikörpers ist.

8. Verwendung nach Anspruch 5, wobei das Empfängerpeptid ein Peptid ist, das sich im Wesentlichen an einen spezifischen Rezeptor des Empfängers bindet.

9. Verwendung eines Retrovirusvektorpartikels mit Empfängerzellenspezifizität, das einen Retrovirusvektor mit einer Antigenbindestelle für einen Antikörper umfasst, der am Hüllprotein des Retrovirusvektors fixiert ist, wobei die Antigenbindestelle des Antikörpers die natürliche Virusrezeptorbindestelle ersetzt, bei der Herstellung eines Medikaments für ein zelltypspezifisches Verfahren, um Gene in vivo unter Verwendung von Retrovirusvektoren in Wirbeltierzellen einzuschleusen.

10. Verwendung nach Anspruch 5 oder 9, wobei das Retroviruspartikel ein Milznekrosevirus ist.

11. Verwendung nach Anspruch 5 oder 9, wobei das Empfängerpeptid oder der Antikörper ein einkettiger Antikörper gegen Haptendinitrophenol ist.

12. Verwendung nach Anspruch 5 oder 9, wobei das Empfängerpeptid eine Antigenbindestelle ist, die sich gegen ein Zelloberflächenprotein der Empfängerzelle richtet.

13. Verwendung nach Anspruch 5 oder 9, wobei das Empfängerpeptid das Rezeptorbindepeptid eines anderen Virus ist.

14. Verwendung nach Anspruch 5 oder 9, wobei der Retrovirusvektor eine Empfängerhülle und eine Wildarthülle ist.

15. Verwendung nach Anspruch 14, wenn er von Anspruch 9 abhängt, wobei die Wildarthülle vom Milznekrosevirus abgeleitet ist.

16. Verwendung nach Anspruch 9 oder 10, 12, 14 oder 15, wenn sie von Anspruch 9 abhängen, wobei der Antikörper ein einkettiger Antikörper anti-Her2neu ist.

## Revendications

1. Utilisation d'un vecteur rétroviral ayant une protéine d'enveloppe dans laquelle une partie du récepteur viral est au moins partiellement définie par un anticorps à chaîne unique et ladite partie du récepteur viral se fixe à un antigène choisi, dans la fabrication d'un médicament à utiliser dans l'infection *in vivo* d'une cellule ayant ledit antigène choisi, dans laquelle ledit médicament est destiné à mettre en contact ledit vecteur rétroviral *in vivo* avec la cellule de telle sorte que ladite particule virale ou une partie de celle-ci soit internalisée dans la cellule, et la cellule ayant l'antigène choisi est infectée par ledit vecteur rétroviral.

2. Utilisation selon la revendication 1 dans laquelle ledit vecteur rétroviral est produit à l'aide d'un plasmide comprenant une séquence nucléotidique codant une seule molécule comprenant une partie d'une protéine d'enveloppe et une partie d'un anticorps à chaîne unique.

3. Utilisation selon la revendication 2 dans laquelle ladite séquence nucléotidique code une partie de la protéine d'enveloppe du virus SNV (Spleen Necrosis Virus).

4. Utilisation selon la revendication 2 dans laquelle le plasmide est celui identifié en figure 2d sous la dénomination pTC5.

5. Utilisation d'un vecteur rétroviral ayant un peptide de ciblage fusionné avec la protéine d'enveloppe dudit vecteur rétroviral pour former une enveloppe de ciblage dans la fabrication d'un médicament destiné à infecter *in vivo* une cellule ayant un antigène choisi, dans laquelle ledit médicament est destiné à mettre en contact ledit vecteur rétroviral *in vivo* avec la cellule de telle sorte que ladite particule virale ou une partie de celle-ci soit internalisée dans la cellule, et la cellule ayant l'antigène choisi est infectée par ledit vecteur rétroviral.

6. Utilisation selon la revendication 5 dans laquelle ledit peptide de ciblage remplace ou perturbe le site naturel de fixation du récepteur viral.

7. Utilisation selon la revendication 5 dans laquelle ledit peptide de ciblage est le site de fixation antigénique d'un anticorps.

8. Utilisation selon la revendication 5 dans laquelle ledit peptide de ciblage est un peptide qui se fixe spécifiquement à un récepteur spécifique de la cible.

9. Utilisation d'une particule de vecteur rétroviral à spécificité de type cellulaire qui comprend un vecteur rétroviral ayant un site de fixation antigénique d'un anticorps fusionné avec la protéine d'enveloppe du vecteur rétroviral, dans laquelle le site de fixation antigénique de l'anticorps remplace le site naturel de fixation du récepteur viral, dans la fabrication d'un médicament destiné à un procédé spécifique de type cellulaire visant à introduire *in vivo* des gènes dans les cellules de vertébrés à l'aide de vecteurs rétroviraux.

10. Utilisation selon la revendication 5 ou 9 dans laquelle la particule rétrovirale est le virus SNV (Spleen Necrosis Virus).

11. Utilisation selon la revendication 5 ou 9 dans laquelle le peptide de ciblage ou anticorps est un anticorps à chaîne unique dirigé contre l'haptène dinitrophénol.

12. Utilisation selon la revendication 5 ou 9 dans laquelle le peptide de ciblage est un site de fixation antigénique dirigé contre une protéine de surface cellulaire de la cellule cible.

13. Utilisation selon la revendication 5 ou 9 dans laquelle le peptide de ciblage est le peptide de fixation du récepteur d'un autre virus.

14. Utilisation selon la revendication 5 ou 9 dans laquelle le vecteur rétroviral comprend une enveloppe de ciblage et une enveloppe de type sauvage.

15. Utilisation selon la revendication 14 lorsqu'elle dépend de la revendication 9 dans laquelle l'enveloppe de type sauvage est dérivée du virus SNV (Spleen Necrosis Virus).

16. Utilisation selon la revendication 9, ou selon la revendication 10, 12, 14 ou 15 lorsqu'elles dépendent de la revendication 9, dans laquelle l'anticorps est un anticorps à chaîne unique anti-Her-2/neu.
